# EUROPÄISCHE PATENTANMELDUNG

(11) **EP 4 151 254 A1**
(43) Veröffentlichungstag der Anmeldung: **22.03.2023**
(21) Anmeldenummer: 21197877.0
(22) Anmeldetag: 21.09.2021
(51) Int. Cl.: A61M 5/32, A61M 5/31, A61M 5/20

(54) **INJEKTIONSGERÄT MIT GERÄTEKAPPE**

(71) Anmelder: Ypsomed AG, 3401 Burgdorf (CH)
(72) Erfinder: Scheurer, Simon, 3006 Bern (CH); Brügger, Martin, 3065 Bolligen (CH); Schrul, Christian, 3400 Burgdorf (CH); Bosshard, Simon Martin, 3006 Bern (CH); Hostettler, Patrick, 3415 Hasle (CH)
(74) Vertreter: Meier Obertüfer, Jürg

(57) **Zusammenfassung**

Die Erfindung betrifft ein Injektionsgerät umfasst eine Gerätekappe zum lösbaren und wiederholten Abdecken einer Injektionsöffnung am distalen Ende eines Reservoir und ein Gerätegehäuse umgebend einen Antriebsmechanismus zum Ausschütten eines Medikaments aus dem Reservoir durch die Injektionsöffnung bei vom Gerätegehäuse abgenommener Gerätekappe. Die Gerätekappe verfügt über einen ersten magnetischen Bereich, auf welchen bei aufgesetzter Gerätekappe eine nach proximal gerichtete und durch einen zweiten magnetischen Bereich des Inj ektionsgeräts erzeugte Kraft wirkt. Durch diese Kraft wird die Gerätekappe wiederholt und zuverlässig in ihre Abdeckposition gezogen oder geschoben, und die aufgesetzte Gerätekappe stabil am Gerätegehäuse gehalten. Auf eine mechanische Kappenhalterung, welche einer Abnutzung durch wiederholte Anwendung unterliegt, kann somit verzichtet werden.

## Beschreibung

### TECHNISCHES GEBIET

Die vorliegende Erfindung betrifft das Gebiet der medizinischen Injektionsgeräte zur Verabreichung von flüssigen Substanzen, insbesondere von Medikamenten oder medizinischen Substanzen wie Insulin- und Hormonpräparationen. Die Erfindung bezieht sich auf ein Injektionsgerät mit einer Gerätekappe zum lösbaren und wiederholten Abdecken einer Injektionsöffnung.

### HINTERGRUND

Unterschiedliche Infusions- und Injektionsgeräte können Patienten bei einer kontrollierten subkutanen oder intramuskulären Verabreichung einer Dosis eines Medikamentes aus einem Reservoir unterstützen. Während bei Infusionssystemen eine Kanüle während längerer Zeit im Körper des Patienten verbleibt werden Injektionsgeräte nach jeder Abgabe wieder von der Injektionsstelle entfernt. In Einweg-Injektionsgeräten ist das Reservoir, beispielsweise eine Fertigspritze, nicht dazu vorgesehen, durch den Patienten ersetzt oder wiederaufgefüllt zu werden. In Mehrweg-Injektionsgeräten dagegen kann das Reservoir, beispielsweise eine Karpule, durch den Patienten wieder aufgefüllt oder ersetzt werden. Ein automatisches Injektionsgerät verfügt über einen Motor oder eine gespannte Feder als Energiequelle zum Antrieb einer Kolbenstange und zur Bewegung eines Kolbens im Reservoir, während bei einer Ausschüttung mit einem manuellen Injektionsgerät die Kolbenstange unmittelbar durch eine Kraft des Patienten bewegt wird.

Diabetes wird oft durch Abgabe von Insulin mittels eines Injektionsgerätes in Form eines Insulin-Pens mit einstellbarer Dosis behandelt. Der Pen verfügt über ein längliches Gehäuse mit einem distalen Ende zur Aufnahme einer Kanüle oder Hohlnadel und mit einem der Injektionsstelle abgewandten proximalen Ende. Ein Insulin-Pen kann als Ein- oder Mehrweg-Pen, automatisch oder manuell betrieben werden. Die abzugebende Insulindosis wird normalerweise eingestellt durch Drehen eines Dosierknopfs und gleichzeitiger Kontrolle einer Dosisanzeige des Insulin-Pens.

Die Patentanmeldung WO 14164444 A1 offenbart einen Verschluss für ein Gefäss, welcher mittels magnetischer Anziehung zwischen über einen Umfang des Verschlusses verteilten Magneten und einem abnehmbaren Kragen des Gefässes an diesem gehalten wird.

Die Patentanmeldung EP 3156088 beschreibt einen Transportkoffer für ein elektrisch betriebenes Injektionsgerät, umfassend eine Temperaturregulierungseinheit und einen Ladeeinheit zum Laden eines Energiespeichers des im Transportkoffer gelagerten Injektionsgeräts.

Die Patentanmeldung WO 18009509 beschreibt einen elektromechanischen Pen mit einer Kappeneinheit und einer Basiseinheit. Die Kappeneinheit umfasst einen Einstech- und Rückzugsmechanismus sowie einen Energiespeicher zum Nachladen einer Batterie der Basiseinheit, welche einen Antrieb der Pens speist. Dadurch wird die Autonomie der Energieversorgung des Antriebs erhöht.

Der Begriff "Medikament" oder "medizinische Substanz" umfasst in diesem Zusammenhang jede fliessfähige medizinische Formulierung, welche geeignet ist zur kontrollierten Verabreichung mittels einer Kanüle oder Hohlnadel, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

### DARSTELLUNG DER ERFINDUNG

Es ist eine Aufgabe der Erfindung ein Injektionsgerät anzugeben, bei welchem eine wiederholt abnehmbare Gerätekappe ohne elastische Schnapper oder sonstige mechanisch belastete Verschleissteile am Gerät befestigt werden kann. Diese Aufgabe wird gelöst durch ein Injektionsgerät mit den Merkmalen von Anspruch 1. Bevorzugte Ausführungsformen der Erfindung sind Gegenstand der abhängigen Ansprüche.

Ein erfindungsgemässes Injektionsgerät umfasst eine Gerätekappe oder Schutzkappe zum lösbaren und wiederholten Abdecken einer Injektionsöffnung am distalen Ende eines Reservoir oder Behältnis und ein Gerätegehäuse umgebend einen Antriebsmechanismus zum Ausschütten eines Medikaments aus dem Reservoir durch die Injektionsöffnung bei vom Gerätegehäuse abgenommener Gerätekappe. Die Gerätekappe verfügt über einen ersten magnetischen Bereich, auf welchen bei aufgesetzter Gerätekappe eine nach proximal gerichtete und durch einen zweiten magnetischen Bereich des Injektionsgeräts erzeugte Kraft wirkt. Durch diese Kraft wird die Gerätekappe wiederholt und zuverlässig in ihre Abdeckposition gezogen oder geschoben, und die aufgesetzte Gerätekappe stabil am Gerätegehäuse gehalten. Auf eine mechanische Kappenhalterung, welche einer Abnutzung durch wiederholte Anwendung unterliegt, kann somit verzichtet werden.

Das Injektionsgerät ist bevorzugt ein tragbares und mobiles Injektionsgerät in Stift- oder Pen-Form mit einer ausgeprägten Längsachse, entlang welcher die Gerätekappe beim Aufsetzen und Abnehmen bewegt wird. Die Gerätekappe ist keine stationäre Basisstation, in welche das Gerätegehäuse nur mit einer bestimmten räumlichen Ausrichtung eingelegt werden kann. Im Falle eines automatischen Antriebs umgibt das Gerätegehäuse eine Antriebseinheit mit einem Antriebsenergiespeicher bevorzugt in Form einer Feder oder eines Elektromotors. Weiter bevorzugt ist das Injektionsgerät ein Mehrweg-Injektionsgerät, bei welchem das entleerte Reservoir alleine oder zusammen mit einem Reservoirhalter ausgetauscht und eine Dosiereinheit mit einem Dosiseinstellmechanismus und gegebenenfalls einem nachladbaren Antriebsenergiespeicher für eine automatische Ausschüttung der Dosis beliebig oft genutzt werden kann.

In vorteilhaften Ausführungsformen umfasst das Injektionsgerät einen Karpulenhalter zum lösbaren Halten des Reservoirs in Form einer vorgefüllten Karpule mit einer durch ein Septum verschlossenen Injektionsöffnung. Die aufgesetzte Gerätekappe deckt den Karpulenhalter und damit die Karpule seitlich oder radial zumindest annähernd über die gesamte axiale Länge ab, so dass diese bei aufgesetzter Gerätekappe nicht sichtbar ist. Die Gerätekappe umgibt nicht nur die Injektionsöffnung und/oder die Injektionsnadel am distalen Ende der Karpule. Weiter bevorzugt ist der zweite magnetische Bereich am Gerätegehäuse angeordnet, und nicht etwa am Karpulenhalter, so dass die Gerätekappe auch ohne den Karpulenhalter am Gerätegehäuse gehalten werden kann.

In einer weiter bevorzugten Ausführungsform umfassen der erste und der zweite magnetische Bereich einen konzentrisch zur Längsachse angeordneten und parallel zur Längsachse magnetisierten Ring mit je einem den ganzen Ringumfang umfassenden Magnetpol an den Stirnseiten. Der magnetische Bereich ist ein flacher Hohlzylinder aus einem permanentmagnetischen Material, mit einer axialen Ausdehnung welche nur einen Bruchteil des Durchmessers beträgt. Alternativ dazu umfassen der erste und der zweite magnetische Bereich je eine Folge von über einen Kreisumfang bevorzugt symmetrisch angeordneten Unterbereichen oder Sektoren mit abwechselnder Magnetisierung. Bei hinreichender Auflösung beziehungsweise grosser Anzahl Sektoren kann die Gerätekappe in einer beliebigen Drehposition aufgesetzt werden.

In einer vorteilhaften Variante sind die magnetischen Bereiche axial und radial so angeordnet, dass sie sich bei aufgesetzter Gerätekappe anziehen. Dazu befindet sich ein magnetischer Nord- oder Südpol des ersten magnetischen Bereichs jederzeit distal eines magnetischen Süd- beziehungsweise Nordpols des zweiten magnetischen Bereichs. Alternativ können sich die beiden magnetischen Bereiche bei aufgesetzter Gerätekappe auch abstossen, insbesondere wenn sich ein Nordpol des ersten Bereichs bei aufgesetzter Gerätekappe sich proximal eines magnetischen Nordpols des zweiten Bereichs befindet. In diesem Fall muss zur vollständigen Entfernung der Gerätekappe ein Kraftmaximum nach der initialen geometrischen Trennung überwunden werden.

Weiter betrifft die Erfindung ein Mehrweg-Injektionsgerät mit einer vom Gerätegehäuse umgebenen Antriebseinheit zum automatischen Ausschütten umfassend einen durch einen Antriebsenergiespeicher in Form einer wieder aufladbaren Batterie gespeisten Elektromotor mit einer Antriebswelle, welche wiederum eine Vortriebshülse oder eine Kolbenstange in Ausschüttrichtung bewegen kann. In der Gerätekappe befindet sich ein Nachladeenergiespeicher als Stromquelle, und an der Gerätekappe sind zwei elektrische Kontakte vorgesehen in elektrisch leitender Verbindung mit dem Nachladeenergiespeicher. Am Gerätegehäuse sind zwei elektrische Kontakte in elektrisch leitender Verbindung mit dem Antriebsenergiespeicher vorgesehen. Die elektrischen Kontakte sind so angeordnet, dass sich je ein Kontakt der Gerätekappe und ein Kontakt des Gehäuses bei aufgesetzter Gerätekappe kontaktieren und ein Laden des Antriebsenergiespeichers durch den Nachladeenergiespeicher ermöglichen. Durch diese Nachlademöglichkeit muss die Batterie im Gerätegehäuse nicht alleine für eine maximale Autonomie dimensioniert werden. Dieses Merkmal und alle darauf aufbauenden Aspekte sind nicht zwingend mit der magnetischen Kopplung der Gerätekappe an das Gerätegehäuse verlinkt, sondern zumindest auch bei einer axialen mechanischen Kopplung anwendbar.

In einer bevorzugten Ausführungsform sind die beiden Kontakte der Gerätekappe und die beiden Kontakte des Gerätegehäuses jeweils um denselben Betrag axial versetzt, so dass sich bei aufgesetzter Gerätekappe der beiden proximalen Kontakte und die beiden distalen Kontakte axial stirnseitig oder radial tangential berühren. Dadurch wird ein unbeabsichtigter Kurzschluss oder eine Kontaktierung der falschen Kontakte zuverlässig verhindert.

In einer weiteren vorteilhaften Ausführungsform fällt einer der elektrischen Kontakte mit einem der magnetischen Bereiche zusammen. Wenn der elektrisch leitfähige Kontakt gleichzeitig magnetisch oder zumindest magnetisierbar ist kann eines der beiden Teile sowie die zugehörige Verbindung mit den Spritzgusskomponenten eingespart werden.

In einer bevorzugten Variante ist der Antriebsenergiespeicher ausschliesslich über die elektrischen Kontakte der Gerätekappe aufladbar, nur Letztere verfügt über einen externen Ladeanschluss zum Nachladen des Injektionsgeräts. Dadurch ist ausgeschlossen, dass das Injektionsgerät während der bestimmungsgemässen Ausschüttung mit einem elektrischen Netz verbunden und der Anwender im Falle eines Isolationsdefekts gefährdet wird.

In einer bevorzugten Variante ist die Gerätekappe eine Funktionserweiterungskappe mit einer Anzeigeeinheit zur Anzeige einer eingestellten Dosis, wobei bevorzugt in diesem Fall das Gerätegehäuse selbst keine Dosisanzeige aufweist. Die Dosiseinstellung kann ebenfalls an der Gerätekappe erfolgen und anschliessend an eine Steuerung des Elektromotors übermittelt werden.

In einer alternativen Ausführungsform eines Mehrweg-Injektionsgeräts mit variabler Dosis und elektromotorgetriebener Ausschüttung kann die Dosiseingabe durch eine berührungsempfindliche Oberfläche am Gerätegehäuse des Injektionsgeräts selbst ermöglicht werden. Dazu führt der Anwender mit einem oder zwei Fingerspitzen eine axiale oder rotative Dosiseinstellbewegung auf der berührungsempfindlichen Oberfläche aus welche durch eine entsprechende Sensorik erkannt und in einen Dosiseinstellwert umgerechnet wird. Zur Erkennung einer Dosierdrehbewegung um die Längsachse ist eine zylindrisch aufgewickelte berührungsempfindliche Folie geeignet. Entsprechend kann auf einen mechanischen Dosiswahlknopf verzichtet werden. Die Anzeige der eingestellten Dosis und/oder einer verbleibenden Medikamentenmenge erfolgt bevorzugt ebenfalls über eine zweidimensionale, aufgerollte Anzeigefolie, idealerweise mittels einer berührungsempfindlichen Anzeigefolie oder Touch-Displays zur integrierten Dosisdrehbewegungserkennung und Dosisanzeige.

### FIGUREN

In Zusammenhang mit den angehängten Figuren werden nachfolgend bevorzugte Ausführungsformen der Erfindung beschrieben. Diese sollen grundsätzliche Möglichkeiten der Erfindung aufzeigen und keinesfalls einschränkend ausgelegt werden. Es zeigen
Fig.1 ein Injektionsgerät mit variabler Dosisabgabe in perspektivischer Ansicht;
Fig.2. einen Axialschnitt im Kontaktbereich zwischen Gerätekappe und Gerätegehäuse;
Fig.3 ein Injektionsgerät mit einer Nachladekappe;
Fig.4 den Axialschnitt aus Fig.2 mit zusätzlich elektrischen Kontakten; und
Fig.5 ein Injektionsgerät mit einer Funktionserweiterungskappe.

### FIGURENBESCHREIBUNG

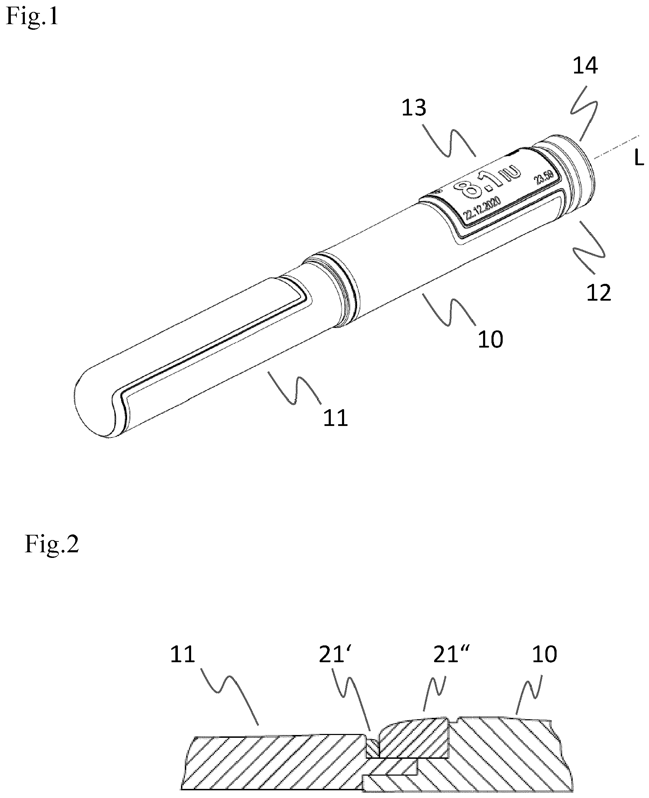

Fig.1 zeigt in Schrägaufsicht ein Injektionsgerät mit variabler Dosisabgabe, umfassend ein längliches Gerätegehäuse 10 mit einer Längsachse L, einen Dosiswahlknopf 12, eine Dosisanzeige 13, und einen Ausschüttknopf 14. Eine aufgesetzte Gerätekappe 11 in Form einer Schutzkappe verdeckt und schützt einen Karpulenhalter und eine Injektionsöffnung umfassend eine auf den Karpulenhalter montierten Injektionsnadel.

Fig.2 zeigt einen Axialschnitt entlang der Längsachse L durch den Kontaktbereich zwischen Gerätekappe 11 und Gerätegehäuse 10 des Injektionsgeräts. Ein erster Magnetring 21' an der Kappe und ein zweiter Magnetring 21" am Gerätegehäuse sind konzentrisch um die Längsachse L vorgesehen und berühren sich über eine kreisringförmige Stirnfläche.

Die beiden Magnetringe 21 sind konzentrisch zur Längsachse angeordnet und parallel zur Längsachse magnetisiert, mit je zwei den ganzen Ringumfang umfassenden magnetischen Bereichen an den Stirnseiten der Ringe. Die Orientierung der Ringe beziehungsweise die Ausrichtung der Magnetpole erfolgt so, dass beispielsweise ein Nordpol an der proximalen Stirnseite des ersten Magnetrings 21' einem Südpol an der distalen Stirnseite des zweiten Magnetrings 21" gegenüberliegt. Die Magnetringe sind bevorzugt als flache Hohlzylinder aus einem Material mit permanentmagnetischen Eigenschaften, beispielsweise aus Ferrit, gefertigt. Zumindest einer der erfindungsgemässen magnetischen Bereiche kann auch aus einem Ferro- oder Paramagnetischen Material mit einer von Null verschiedenen magnetischen Permeabilität µ gefertigt sein, welches im Feld eines Permanentmagneten magnetisiert und durch diesen angezogen wird. Magnetische Bereiche in Ringform können als separate, insbesondere metallische, Komponenten gefertigt und anschliessend einfach auf einen Sitz der spritzgussgefertigten Gerätekappe und/oder des Gerätegehäuses aufgepresst, geklebt oder geschnappt werden. Alternativ kann ein magnetischer Bereich aus magnetisierbarem Kunststoff in einem zweikomponenten-Spritzgussverfahren zusammen mit den nichtmagnetisierbaren Bereichen gefertigt werden.

Die Magnetringe können auch über den Umfang verteilt je eine Abfolge von unterschiedlich oder gegensinnig magnetisierten Bereichen umfassen, beispielsweise acht Sektoren mit je einem Winkelbereich von 45°. Bei ausreichend kleinen Winkelbereichen kann die Kappe praktisch in jeder Drehposition relativ zum Gerätegehäuse auf Letzteres aufgesetzt werden. Die magnetischen Bereiche können auch durch nichtmagnetische Bereiche getrennt sein und beispielsweise nur vier diskrete Pole aufweisen, wobei durch geometrische Führungsmittel die rotative Ausrichtung gewährleistet wird. Die Magnetringe brauchen nicht zwingend an der Peripherie von Kappe und Gerätegehäuse angeordnet zu sein, sondern können auch leicht radial nach innen versetzt, durch einen proximalen Mantelabschnitt der Gerätekappe und einen distalen Mantelabschnitt des Gehäuses abgedeckt und somit für den Anwender nicht sichtbar sein. Durch eine optimierte Anordnung und Dimensionierung der magnetischen Bereiche ergibt sich für den Anwender ein angenehmes und qualitativ taktil hochwertiges Gefühl beim Aufsetzen und Abziehen der Gerätekappe.

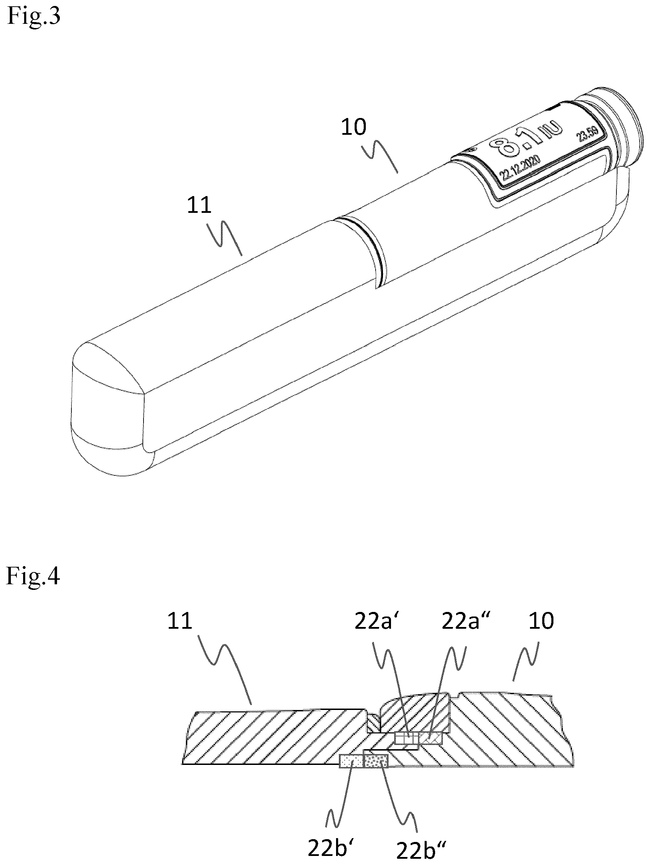

Fig.3 zeigt ein Injektionsgerät in Form eines elektromechanischen Pens mit einer aufgesetzten Gerätekappe 11 in Form einer Nachladekappe, welche eine Stromquelle in Form einer Batterie oder eines Akkumulators als Nachladeenergiespeicher umfasst zum wiederholten Nachladen eines Antriebsenergiespeichers im Gerätegehäuse 10 des Injektionsgeräts. Die Gerätekappe umfasst einen seitlichen Aufnahmebereich ähnlich der Schutzkappe aus Fig.1, zur Aufnahme und magnetischen Fixierung des Gerätegehäuses 10 mit oder ohne Karpulenhalter. Neben dem Aufnahmebereich befindet sich über die gesamte Länge verteilt der Nachladeenergiespeicher.

Fig.4 zeigt einen Axialschnitt entlang der Längsachse L durch den Kontaktbereich zwischen Gerätekappe 11 aus Fig.3 und dem Gerätegehäuse 10 des Injektionsgeräts. Die Gerätekappe 11 umfasst einen ersten 22a' und einen zweiten elektrischen Kontakt 22b' welche mit einem ersten 22a" beziehungsweise einem zweiten 22b" elektrischen Kontakt des Gerätegehäuses 10 eine elektrisch leitende Verbindung bilden. Dadurch wird ein Ladekreis gebildet, über welchen Antriebsenergiespeicher des Injektionsgeräts 10 durch den Nachladeenergiespeicher der Gerätekappe 11 nachgeladen werden kann. Die zwei Kontakte 22a', 22b' der Kappe und die zwei Kontakte 22a", 22b" des Gehäuses sind jeweils um denselben Betrag axial versetzt, so dass bei aufgesetzter Kappe die ersten, proximalen Kontakte 22a', 22a" und die zweiten, distalen Kontakte 22b', 22b" je einander axial stirnseitig oder radial tangential kontaktieren.

Bevorzugt sind die gerätegehäuseseitigen Kontakte 22" die ausschliesslichen Ladekontakte des Antriebsenergiespeichers, es ist also kein weiterer Anschluss am Gerätegehäuse vorhanden und der Antriebsenergiespeicher ist auch nicht zum Austausch vorgesehen. Weiter bevorzugt sind die elektrischen Kontakte rotationssymmetrisch um die Geräteachse L ausgebildet, oder sie umfassen diskrete Kontaktsektoren mit einem minimalen umfangmässigen Überlapp, so dass an die rotatorische Ausrichtung von Gerätekappe 11 und Gerätegehäuse 10 zur elektrischen Kontaktierung keine Anforderungen gestellt werden. Alternativ können durch geeignete mechanische oder magnetische Führungsmittel die rotatorische Ausrichtung von Gerätekappe 11 und Gerätegehäuse 10 bestimmt sein, wodurch die elektrischen Kontakte ohne Kurzschlussgefahr axial auf gleicher Höhe sowie radial und/oder über den Umfang separiert vorgesehen sein können. Zumindest einer der beiden ersten beziehungsweise zweiten elektrischen Kontakte kann zu Toleranzausgleichszwecken auch gefedert gelagert sein.

Ein elektrischer Antriebsenergiespeicher mit einer Kapazität von 70mAh ist beispielsweise ausreichend um nacheinander fünf Karpulen mit jeweils 1.6m1 auszuschütten. Mit einem Nachladeenergiespeicher derselben Grössenordnung könnte die Autonomie des Injektionsgeräts also auf zehn Karpulen verdoppelt werden. Ein solcher Speicher kann zwischen Karpulenhalter und Kappenhülle im Innern einer Gerätekappe wie in Fig.1 dargestellt untergebracht werden, deren Durchmesser denjenigen des Gerätegehäuses nicht oder höchstens marginal übertrifft. Der Nachladeenergiespeicher weist aber bevorzugt eine Speicherkapazität auf welche diejenige des Antriebsenergiespeichers um ein Vielfaches übersteigt. Die Autonomie des Injektionsgeräts wird durch das damit ermöglichte wiederholte Nachladen zwar vervielfacht, die dazu erforderliche Gerätekappe beeinflusst dafür die Abmessungen und den Formfaktor des Injektionsgeräts deutlich, beispielsweise so wie die in Fig.3 dargestellte Nachladekappe.

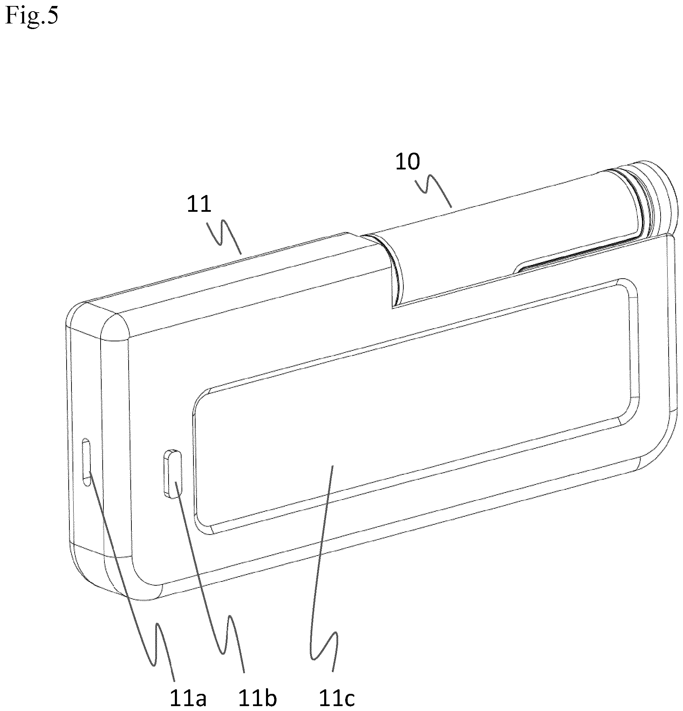

Fig.5 zeigt Injektionsgerät 10 in Form eines elektromechanischen Pens mit einer aufgesetzten Gerätekappe 11 in Form einer Funktionserweiterungskappe, ebenfalls mit einem Nachladeenergiespeicher zum wiederholten Nachladen eines Antriebsenergiespeichers im Gerätegehäuse 10 des Injektionsgeräts. Der Nachladeenergiespeicher der Gerätekappe 11 seinerseits wird über einen Ladeanschluss 11a in Form eines USB-Steckers über ein elektrisches Verteilnetz oder eine weitere Speichereinheit aufgeladen. Die Gerätekappe 11 verfügt weiter über einen Steuerknopf 11b und eine Anzeigeeinheit 11c zur Übermittlung von Informationen oder Instruktion von Anwendungsschritten. Gegenüber der Nachladekappe aus Fig.3 ist die Funktionserweiterungskappe in einer Richtung senkrecht zur Längsachse noch weiter ausgedehnt, passt aber mit seinem flachen Formfaktor weiterhin zumindest in jede Jackentaschen.

Alternativ zu einem galvanischen Ladeanschluss kann der Nachladeenergiespeicher auch induktiv aufgeladen werden. Der Steuerknopf 11b ist Teil einer optionalen Eingabeeinheit mit mehreren berührungsempfindlichen Elementen oder dient auch nur zum Ein-/Ausschalten der Funktionserweiterungskappe. Um das Gerätegehäuse, beziehungsweise das Injektionsgerät ohne die Funktionserweiterungskappe, möglichst klein und diskret zu halten können weitere Funktionen exklusiv von der Funktionserweiterungskappe übernommen werden. Beispielsweise erfolgt dazu die Einstellung und Anzeige der auszuschüttenden Dosis über eine Dosiswahleinheit und die Anzeigeeinheit 11c der Gerätekappe, bevorzugt kombiniert über eine berührungsempfindliche Anzeigeeinheit. In diesem Fall verfügt das Gerätegehäuse selbst weder über Dosisanzeige noch Dosiswahlknopf, und die gewählte Dosis wird drahtlos, beispielsweise mittels NFC, Bluetooth, oder codierenden Lichtsignalen, oder über eine Steckverbindung, an eine Steuerung des Elektromotors übermittelt. Als Kompromiss wird die abzugebende Dosis mit dem Dosiswahlknopf am Gerätegehäuse eingestellt aber auf der Anzeigeeinheit 11c der Gerätekappe angezeigt. In diesen Fällen muss das Gerätegehäuse für jede Dosiseinstellung mit der Funktionserweiterungskappe gekoppelt werden, entsprechend kann die Kapazität des Antriebsenergiespeichers weiter reduziert werden bis zu einem Wert für die Abgabe einer Maximaldosis. Neben einer Batterie kommt damit auch ein Kondensator als Antriebsenergiespeicher in Frage. Ausgelöst wird die Ausschüttung durch einen manuell zu betätigenden Auslöseknopf am proximalen Ende und/oder selbsttätig unmittelbar nach Erkennen der erfolgten Aufsetzbewegung des Injektionsgeräts auf die Injektionsstelle.

Weiter betrifft die Erfindung ein Mehrweg-Injektionsgerät mit einer vom Gerätegehäuse umgebenen Antriebseinheit zum automatischen Ausschütten umfassend einen Antriebsenergiespeicher in Form einer gespannten Spiralfeder zur Rotation einer Antriebswelle. Das mechanische Spannen oder Nachladen der Spiralfeder geschieht beim Einstellen der Dosis durch Drehen des Dosiswahlknopfs. Ein derartiges, minimal elektronisch ausgestaltetes Injektionsgerät umfasst weiter eine Empfangseinheit zum drahtlosen Empfangen eines Dosiswerts einer abzugebenden Dosis, und eine optische und/oder akustische Anzeige, beispielsweise eine ampelfarbige LED zur Erleuchtung eines um das Injektionsgerät umlaufenden, ringförmigen Lichtleiters. Die optische Anzeige leuchtet auf zur allseitigen Signalisierung dass eine am Dosiswahlknopf aktuell eingestellte Dosis einem empfangenen Dosiswert entspricht, alternativ und/oder ergänzend signalisiert die Anzeige eine nicht korrekt eingestellte Dosis. Der besagte Dosiswert wird vorgängig in der Funktionserweiterungskappe oder in einem mit dem Injektionsgerät drahtlos verbundenen mobilen Gerät berechnet, bevorzugt auf Basis von aktuellen physiologischen Messwerten wie beispielsweise einem Blutzuckerwert des Anwenders, und an die Empfangseinheit übermittelt.

**BEZUGSZEICHENLISTE**

| | | | |
|---|---|---|---|
| 10 | Gerätegehäuse | 21 | Magnetischer Kontakt |
| 11 | Gerätekappe | 22 | Elektrischer Kontakt |
| 12 | Dosiswahlknopf | 11a | Ladeanschluss |
| 13 | Dosisanzeige | 11b | Steuerknopf |
| 14 | Ausschüttknopf | 11c | Anzeigeeinheit |

## Patentansprüche

1. Injektionsgerät mit einer Gerätekappe (11) zum lösbaren Abdecken einer Injektionsöffnung am distalen Ende eines Reservoirs und mit einem Gerätegehäuse (10) umfassend einen Antriebsmechanismus zum Ausschütten eines Medikaments aus einem Reservoir durch die Injektionsöffnung bei vom Gerätegehäuse (10) abgenommener Gerätekappe (11), **dadurch gekennzeichnet, dass** die Gerätekappe (11) über einen ersten magnetischen Bereich (21') verfügt, auf welchen bei aufgesetzter Gerätekappe eine nach proximal gerichtete und durch einen zweiten magnetischen Bereich (21") des Injektionsgeräts erzeugte Kraft wirkt.

2. Inj ektionsgerät nach Anspruch 1, **dadurch gekennzeichnet, dass** das Inj ektionsgerät einen Karpulenhalter umfasst zum lösbaren Halten einer Karpule relativ zum Gerätegehäuse (10), wobei die aufgesetzte Gerätekappe (11) den Karpulenhalter seitlich zumindest annähernd vollständig abdeckt.

3. Injektionsgerät nach Anspruch 2, **dadurch gekennzeichnet dass** der zweite magnetische Bereich (21") am Gerätegehäuse (10) angeordnet ist.

4. Injektionsgerät nach einem der Ansprüche 1 bis 3, **dadurch gekennzeichnet dass** der erste (21') und/oder der zweite (21") magnetische Bereich einen konzentrisch zur Längsachse angeordneten und parallel zur Längsachse magnetisierten Ring umfassen.

5. Injektionsgerät nach einem der Ansprüche 1 bis 3, **dadurch gekennzeichnet dass** der erste und/oder der zweite magnetische Bereich eine Folge von über einen Kreisumfang angeordneten Unterbereichen mit abwechselnder Magnetisierung umfassen.

6. Injektionsgerät nach Anspruch 4 oder 5, **dadurch gekennzeichnet dass** sich unterschiedliche Magnetpole des ersten und des zweiten magnetischen Bereichs bei aufgesetzter Gerätekappe axial gegenüberliegen und anziehen.

7. Injektionsgerät nach einem der vorhergehenden Ansprüche, wobei der Antriebsmechanismus einen durch einen Antriebsenergiespeicher gespeisten Elektromotor umfasst, wobei an der Gerätekappe (11) ein erster (22a') und ein zweiter (22b') elektrischer Kontakte und am Gerätegehäuse (10) ein erster (22a") und ein zweiter (22b") elektrischer Kontakt derart vorgesehen sind, dass sich der erste Kontakt (22a') der Gerätekappe und der erste Kontakt (22a") des Gerätegehäuses bei aufgesetzter Gerätekappe kontaktieren und ein Laden des Antriebsenergiespeichers durch eine Stromquelle der Gerätekappe ermöglichen.

8. Injektionsgerät nach Anspruch 7, **dadurch gekennzeichnet dass** die beiden Kontakte der Gerätekappe und die beiden Kontakte des Gerätegehäuses jeweils um denselben Betrag axial versetzt sind und bei aufgesetzter Gerätekappe der distale Kontakt (22b') der Gerätekappe den distalen Kontakt (22b") des Gerätegehäuses elektrisch kontaktiert.

9. Injektionsgerät nach Anspruch 7, **dadurch gekennzeichnet, dass** ein elektrischer Kontakt der Gerätekappe und/oder ein elektrischer Kontakt des Gerätegehäuses identisch ist mit dem ersten (21') beziehungsweise dem zweiten magnetischen Bereich (21").

10. Injektionsgerät nach Anspruch 7, **dadurch gekennzeichnet dass** der Antriebsenergiespeicher ausschliesslich über die elektrischen Kontakte (22a', 22b') der Gerätekappe (11) aufladbar sind.

11. Injektionsgerät nach Anspruch 7, **dadurch gekennzeichnet dass** die Gerätekappe eine Anzeigeeinheit (11c) aufweist zur Anzeige einer eingestellten Dosis.

12. Injektionsgerät nach Anspruch 7, **dadurch gekennzeichnet, dass** das Gerätegehäuse eine berührungsempfindliche Oberfläche aufweist zur Erkennung einer Dosiseinstellbewegung durch den Anwender.
